# EUROPÄISCHE PATENTANMELDUNG

(11) **EP 4 268 868 A1**
(43) Veröffentlichungstag der Anmeldung: **01.11.2023**
(21) Anmeldenummer: 22170084.2
(22) Anmeldetag: 26.04.2022
(51) Int. Cl.: A61M 15/08, A61M 15/00, A61M 11/00, B05B 11/00

(54) **SPENDER UND AUSWERTEEINHEIT HIERFÜR**

(71) Anmelder: Aptar Radolfzell GmbH, 78315 Radolfzell (DE)
(72) Erfinder: Helmlinger, Michael, 78315 Radolfzell-Böhringen (DE); Kohnle, Jörg, 78056 Villingen-Schwenningen (DE)
(74) Vertreter: Witte, Weller und Partner Patentanwälte mbB Stuttgart

(57) **Zusammenfassung**

Bekannt ist ein Spender (10) zum Austrag pharmazeutischer oder kosmetischer Medien mit einem Gehäuse (12), einem Medienspeicher (14) sowie einer Austragöffnung (16). Bekannt ist es weiterhin, einen solchen Spender mit einer elektronischen Auswerteeinheit (40) zur Erfassung der Nutzung des Spenders zu versehen, die zur lösbaren Anbringung am Gehäuse (12) des Spenders (10) ausgebildet ist.

Es wird vorgeschlagen, dass an der Außenseite des Gehäuses (12), insbesondere an einem Speicherkörper (18) des Gehäuses (12), eine magnetische oder magnetisierbare Haltefläche (70) vorgesehen ist. Eine Anlageseite (42) der Auswerteeinheit (40) ist hierzu korrespondierend magnetisch oder magnetisierbar ausgebildet, so dass die Auswerteeinheit (40) magnetisch am Gehäuse (12) befestigt werden kann.

Alternativ wird vorgeschlagen, dass die Auswerteeinheit (40) mindestens zwei und vorzugsweise mindestens drei elastisch auslenkbare Haltearme (80, 82) aufweist, die das Gehäuse (12), insbesondere einen Speicherkörper (18) des Gehäuses, umgreifen und damit einen klemmenden Halt der Auswerteinheit (40) am Gehäuse bewirken.

## Beschreibung

### ANWENDUNGSGEBIET UND STAND DER TECHNIK

Die Erfindung betrifft einen Spender zum Austrag pharmazeutischer oder kosmetischer Medien sowie insbesondere eine elektronische Auswerteeinheit für einen solchen Spender.

Ein gattungsgemäßer Spender dient dem Austrag von Medien, insbesondere von pharmazeutischen Flüssigkeiten. Es ist bekannt, solche Spender mit Auswerteeinheiten zu versehen, die über eine Sensorik die Nutzung des Spenders erfassen, beispielsweise um eine nachfolgende Prüfung zu gestatten, ob ein Medikament in richtiger Weise und in Übereinstimmung mit einem Medikationsplan eingenommen wird.

Bekannte Auswerteeinheiten sind in den jeweiligen Spender baulich integriert oder sind lösbar am Spender angebracht, so dass sie vom Spender gelöst und an einen anderen Spender gleichen Typs angebracht werden können.

Bei lösbaren Auswerteeinheiten ist ein Kopplungsmechanismus erforderlich, um sie am Gehäuse des Spenders anzubringen.

### AUFGABE UND LÖSUNG

Aufgabe der Erfindung ist es, eine Auswerteeinheit für einen Spender zur Verfügung zu stellen, die eine bequeme Handhabung im Zuge der Demontage und Montage an einem Spender gestattet.

Erfindungsgemäß wird ein Spender zum Austrag pharmazeutischer oder kosmetischer Medien vorgeschlagen, der über eine elektronische Auswerteeinheit verfügt. Die Erfindung betrifft darüber hinaus auch die Auswerteeinheit als solche.

Ein erfindungsgemäßer Spender weist ein Gehäuse auf, das die Außenkontur des Spenders bildet und mehrteilig ausgebildet sein kann. Insbesondere kann ein solches Gehäuse einen Speicherkörper aufweisen, der den Medienspeicher und insbesondere einen Flüssigkeitsspeicher bildet, in dem das Medium, insbesondere eine pharmazeutische Flüssigkeit, vor dem Austrag gelagert ist.

Der Spender weist eine Austragöffnung auf, durch die Medium aus dem Medienspeicher in eine umgebende Atmosphäre abgegeben werden kann.

Bevorzugt ist der Spender zur Ausbringung von Flüssigkeit ausgebildet, insbesondere in Form eines Strahls, eines Sprühstrahls, oder in Form von Einzeltropfen. Stattdessen kann der Spender jedoch auch für andere Medien vorgesehen sein, so insbesondere zur Ausbringung von Pulver zum Zwecke der Inhalation.

Ist der Spender als Flüssigkeitsspender ausgebildet, so kann es sich insbesondere um einen Pumpspender handeln, der über eine manuell betätigbare Pumpeinrichtung verfügt, die Flüssigkeit aus dem Flüssigkeitsspeicher zur Austragöffnung fördert. Alternativ handelt es sich um einen Quetschflaschenspender, der einen elastisch verformbaren Speicherkörper aufweist, durch dessen manuelle Komprimierung Flüssigkeit ausgetragen werden kann.

Der Spender kann weiterhin als Inhalator ausgebildet sein, insbesondere als Spender mit einem zur Abgabe einer Dosis der Flüssigkeit niederdrückbarem ContainerzurAbgabe einer definierten Dosis des Medikaments. Gemäß einem weiteren Beispiel kann der Spender als Nasalspender ausgebildet sein, insbesondere mit einem langgestreckten Nasalapplikator, an dessen distalem Ende die Austragöffnung vorgesehen ist.

Die elektronische Auswerteeinheit ist zur Erfassung der Nutzung des Spenders ausgebildet. Sie weist hierfür elektrische bzw. elektronische Komponenten auf, insbesondere eine Batterie, einen Prozessor und mindestens einen Sensor. Über den Sensor werden Daten erfasst, die Rückschlüsse auf die Spendernutzung zulassen. Die Sensoren können dabei dafür ausgebildet sein, unmittelbar eine externe Kraftbeaufschlagung zu erfassen, wie sie beispielsweise beim Niederdrücken einer Betätigungshandhabe zur Durchführung einer Pumpbetätigung auftritt. Vorzugsweise ist jedoch mindestens ein Sensor vorgesehen, der berührungslos und ohne externe Kraftbeaufschlagung Daten erfassen kann, wobei es sich insbesondere um ein Mikrofon und/oder einen Bewegungssensor handeln kann. Beispielsweise können die Ausgangsdaten eines Mikrofons ausgewertet werden, um charakteristische Geräusche während der Nutzung zu erkennen.

Die Auswerteeinheit umfasst vorzugsweise eine Ausgabeeinrichtung, insbesondere in Form mindestens einer LED, eines Displays, eines Vibrators und/oder eines Lautsprechers, um Signale an den Nutzer abzugeben, beispielsweise um die Information zu übermitteln, dass ein Austrag sensiert worden ist oder gemäß Medikationsplan ansteht.

Die Auswerteeinheit ist vorzugsweise dafür ausgebildet, zusammen mit einem externen Gerät wie insbesondere einem Smartphone verwendet zu werden. Zur Kommunikation mit dem externen Gerät weist die Auswerteeinheit vorzugsweise ein Funkmodul, insbesondere ein Bluetooth- und/oder WIFI-Funkmodul oder aber ein GSM-/3G-/4G- oder 5G-Funkmodel auf.

Um die Auswerteeinheit schnell und bequem vom Gehäuse des Spenders trennen und an einen neuen Spender ankoppeln zu können, wird gemäß einem ersten Aspekt der Erfindung vorgeschlagen, dass an der Außenseite des Gehäuses des Spenders, insbesondere an einem Speicherkörper des Gehäuses, eine magnetische oder magnetisierbare Haltefläche vorgesehen ist. Korrespondierend hierzu ist eine Anlageseite der Auswerteeinheit magnetisch oder magnetisierbar ausgebildet, so dass die Auswerteeinheit magnetisch am Gehäuse befestigt werden kann.

Die Anbringung der Auswerteeinheit ist durch die erfindungsgemäß vorgesehene Magnetverbindung sehr einfach und bequem möglich. Sobald die Auswerteeinheit in der Bereich des Gehäuses gebracht wird, der für die Anbringung der Auswerteeinheit vorgesehen ist, wird die Auswerteeinheit magnetisch angezogen und bleibt in der bestimmungsgemäßen Position am Gehäuse des Spenders haften.

Neben der Bequemlichkeit der Handhabung wird hierdurch bei geeigneter Gestaltung und insbesondere Größe der Haltefläche am Gehäuse auch erreicht, dass die Auswerteeinheit recht genau eine vordefinierte Position einnimmt. Dies stellt einen Vorteil dar, denn diese vordefinierte Position kann so gewählt sein, dass sie zur Erkennung der Nutzung des Spenders, insbesondere über einen Bewegungssensor oder ein Mikrofon, besonders gut geeignet ist, dass also die über den mindestens einen Sensor erfassbaren Daten, bspw. Geräusche während der Nutzung, einen sicheren Rückschluss auf die Nutzung des Spenders zulassen.

Vorzugsweise ist am Gehäuse eine magnetisierbare und insbesondere eine metallische Haltefläche vorgesehen, während an der Auswerteeinheit mindestens ein Permanentmagnet vorgesehen ist, aber auch die umgekehrte Konstellation ist möglich. Auch ist eine Gestaltung möglich, bei der sowohl die Haltefläche als auch die magnetische Gestaltung der Auswerteeinheit mit Permanentmagneten realisiert sind.

Als Permanentmagneten kommen insbesondere Neodym-Magneten in Frage, da solche Magneten eine starke Magnetkraft bereitstellen und daher für die sichere Befestigung der Auswerteeinheit am Gehäuse des Spenders besonders von Vorteil sind.

Die am Gehäuse vorgesehene Haltefläche kann auf verschiedene Arten bereitgestellt werden. Insbesondere bevorzugt ist es, wenn die Haltefläche im Bereich eines beschrifteten Etiketts vorgesehen ist. Sie kann unmittelbar das Etikett bilden oder mittels des Etiketts an einer Gehäusefläche des Gehäuses festgeklebt sein. Das Etikett kann die Produktbezeichnung oder eine Produktbeschreibung zu dem im Medienspeicher enthaltenen pharmazeutischen oder kosmetischen Produkt umfassen. Alternativ kann das Etikett auch eine Handhabungsanleitung enthalten, die die Anbringung der Auswerteeinheit beschreibt. Diese kann auch beispielsweise in einem Symbol bestehen, welches die Anbringung verdeutlicht.

Die Haltefläche kann auch unabhängig vom Etikett selbstständig am Gehäuse mittels einer Klebeverbindung angebracht sein. Auch sind Gestaltungen möglich, bei denen die Haltefläche durch eine Haltehülse oder Halteklemme gebildet wird, die klemmend am Gehäuse des Spenders angebracht ist. Eine solche Haltehülse oder Halteklemme kann insbesondere vollständig oder nahezu vollständig metallisch sein. Sie kann jedoch auch aus Kunststoff gefertigt sein und einen magnetischen oder magnetisierbaren Einsatz aufweisen.

Gemäß einem zweiten Aspekt der Erfindung wird eine alternative Gestaltung vorgeschlagen, die die bequeme Anbringung der Auswerteeinheit am Gehäuse des Spenders gestattet. Gemäß dieser alternativen Gestaltung ist vorgesehen, dass die Auswerteeinheit mindestens zwei und vorzugsweise mindestens drei elastisch auslenkbare Haltearme aufweist, die das Gehäuse umgreifen und damit einen klemmenden Halt der Auswerteinheit am Gehäuse bewirken.

Die Haltearme erstrecken sich vorzugsweise in unterschiedlichen Richtungen von einem Gehäuse der Auswerteeinheit um das Spendergehäuse herum. Die Haltearme definieren im entspannten Zustand einen lichten Querschnitt, der kleiner als der Querschnitt des Gehäuses und insbesondere des Speicherkörpers ist, so dass sie nach Anbringung am Gehäuse unter elastischer Spannung stehen. Zur Einbringung des Gehäuses in den lichten Querschnitt werden die Haltearme vorzugsweise aufgebogen. Dies kann unmittelbar manuell an den Haltearmen erfolgen oder durch mit den Haltearmen verbundene Greifflächen geschehen, die bei Kraftbeaufschlagung mittelbar die Haltearme öffnen und den lichten Querschnitt dadurch vergrößern.

Die Haltearme lassen sich flexibel auslenken, so dass sie eine Kopplung an Speicherkörper unterschiedlicher Querschnittsform und insbesondere unterschiedlicher Durchmesser gestatten.

Die einzelnen Haltearme sind vorzugsweise gekrümmt. Insbesondere erstrecken sie sich kreisabschnittsförmig, um einen in etwa kreiszylindrischen Gehäuseabschnitt zu umgreifen, insbesondere einen entsprechend geformten Speicherkörper. Die Haltearme erstrecken sich vorzugsweise über einen Winkelbereich von mindestens 120°, so dass zwei in entgegengesetzte Richtung das Gehäuse umgebende Haltearme das Gehäuse um mindestens etwa 240° umgreifen. Der Durchmesser des Grundkreises der Kreisabschnittsform, entsprechend derer die Haltearme geformt sind, beträgt vorzugsweise zwischen 10 mm und 25 mm.

Die Zahl der Haltearme beträgt vorzugsweise mindestens drei, wobei von diesen drei Haltearmen vorzugsweise zwei sich in einer ersten Erstreckungsrichtung um das Spendergehäuse herum erstrecken, während der dritte Haltearm zwischen diesen beiden Haltearmen angeordnet ist und sich in entgegengesetzter Erstreckungsrichtung um das Spendergehäuse herum erstreckt.

Unabhängig von der Anbringungsmethode über Magnetismus oder über die Haltearme kann die Auswerteeinheit ein Gehäuse aufweisen, welches nicht-modular aufgebaut ist und für den Anwender nicht weiter bestimmungsgemäß zerlegbar ist. Eine besondere Ausgestaltung eines erfindungsgemäßen Spenders und einer erfindungsgemäßen Auswerteeinheit sieht jedoch vor, dass die Auswerteeinheit eine Befestigungseinheit und eine Elektronikeinheit umfasst, die getrennte Baueinheiten darstellen.

Die Befestigungseinheit ist dabei zur lösbaren Anbringung am Gehäuse ausgebildet und weist hierfür die beschriebene magnetische oder magnetisierbare Gestaltung und/oder die beschriebenen Haltearme auf. Die hiervon trennbare Elektronikeinheit ist zur lösbaren Anbringung an der Befestigungseinheit vorgesehen. Die Elektronikeinheit umfasst vorzugsweise die Gesamtheit der elektronischen Komponenten der Auswerteeinheit, mindestens jedoch die Batterie, den Prozessor sowie mindestens einen Sensor.

Die Zweiteilung der Auswerteeinheit gestattet es, die Elektronikeinheit mit unterschiedlichen Befestigungseinheiten zu verwenden, beispielsweise solchen zur magnetischen Befestigung am Spendergehäuse und solchen zur Befestigung am Spendergehäuse mit oben beschriebenen Haltearmen.

Die gleiche Elektronikeinheit kann demnach durch Konfiguration mit unterschiedlichen Befestigungseinheiten zur Anbringung an vielen unterschiedlichen Spendern vorgesehen sein.

Die Möglichkeit, die Befestigungseinheit und die Elektronikeinheit zu trennen und in anderer Konfiguration wieder zusammenzusetzen, muss nicht zwingend für den Endanwender gegeben sein. Die genannte Modularität kann auch dafür genutzt werden, um einheitliche Elektronikeinheiten bereits im Rahmen der Herstellung und Bereitstellung bedarfsgerecht mit passenden Befestigungseinheiten zu versehen und ggf. fest zu verbinden.

Besonders bevorzugt ist es allerdings, wenn die Befestigungseinheit zur werkzeuglos lösbaren Anbringung an der Elektronikeinheit ausgebildet ist und damit dem Endanwender selbst die Möglichkeit des Wechsels der Befestigungseinheit offensteht. Insbesondere kann zu diesem Zweck vorgesehen sein, dass die Elektronikeinheit magnetisch an der Befestigungseinheit befestigt ist oder die Elektronikeinheit durch einen Rastmechanismus mit einer elastisch auslenkbaren Rastlasche an der Befestigungseinheit befestigt ist.

Die Befestigungseinheit ist vorzugsweise frei von elektronischen Komponenten. Sie kann jedoch auch Träger von Sensoren sein und/oder ein elektronisch auslesbares Identifikationsmerkmal aufweisen, beispielsweise ein Identifikationsmerkmal, das in einem RFID-Chip der Befestigungseinheit abgelegt ist und durch einen Sensor der Elektronikeinheit auslesbar ist. Hierdurch kann protokolliert werden, mit welcher Befestigungseinheit die Elektronikeinheit verbunden ist.

Besonders bevorzugt ist es, wenn die elektronische Auswerteeinheit eine Anlageseite aufweist, die im angebrachten Zustand der Auswerteeinheit in Richtung des Gehäuses weist, wobei an der Anlageseite mindestens zwei zueinander parallele Anlagerippen zur Anlage am Gehäuse vorgesehen sind.

Vorzugsweise handelt es sich um genau zwei oderdrei zueinander parallele Anlagerippen, wobei die einzelnen Anlagerippen auch unterbrochen sein können und aus zueinander fluchtenden Rippensegmenten bestehen können. Die Rippen bilden erhabene Bereiche zur Anlage am Gehäuse. Die Rippen weisen vorzugsweise eine Länge zwischen 10 mm und 30 mm auf. Die Breite der Rippen beträgt vorzugsweise 5 mm oder weniger, insbesondere vorzugsweise 3 mm oder weniger. An ihrer zum Gehäuse weisenden Seite sind die Rippen vorzugsweise mit einer ausgerundeten Kante versehen.

Zwischen den mindestens zwei Anlagerippen ist ein zurückgesetzter Zwischenbereich vorgesehen, der die Anlagerippen voneinandertrennt. In diesem Zwischenbereich ist die Anlageseite der Auswerteeinheit derart zurückgesetzt, so dass sie nicht am Gehäuse des Spenders anliegt. Der Kontakt zwischen der Anlageseite und dem Gehäuse des Spenders beschränkt sich demnach auf die mindestens zwei Anlagerippen.

Eine Auswerteeinheit mit solchen Anlagerippen gestattet eine sichere Anlage am Gehäuse des Spenders, da die Andruckkraft in definierten Anlagebereichen stattfindet. Zudem gestatten die Anlagerippen die Anlage an Gehäusen unterschiedlicher Maße und insbesondere Speicherkörper mit unterschiedlich gewölbterAußenkontur.

Welche Krümmungsradien solcher Außenkonturen jeweils abgedeckt werden, hängt von der Geometrie der Anlageseite ab. Bevorzugt ist eine Gestaltung, bei der die Anlagerippen einen Abstand voneinander von mindestens 8 mm aufweisen, wobei sich die Anlagerippen vorzugsweise um mindestens 1 mm, vorzugsweise um mindestens 1,5 mm über die Anlagefläche in diesem Zwischenbereich erheben. Zwei Anlagerippen, zwischen denen ein Zwischenbereich von 8 mm vorgesehen ist, der 1 mm zurückgesetzt ist, können an Gehäuseabschnitten mit einem Krümmungsradius größer 10 mm angebracht werden, ohne dass der Gehäuseabschnitt abseits derAnlagerippen in Kontakt mit der Anlageseite gelangt. Mit einem Zwischenbereich von 8 mm Weite, der 1,5 mm zurückgesetzt ist, ist die Anbringung bis zu einem Krümmungsradius von ca. 6 mm möglich.

Eine oder mehrere der Anlagerippen können an einem elastischen Ausleger angebracht sein, insbesondere an einem Ausleger in Form einer an drei Seiten freigeschnittenen Kunststoffzunge, die ein federndes Verhalten orthogonal zur Anlageseite ermöglicht. Es kann auch vorgesehen sein, dass eine oder mehrere, nicht aber alle Anlagerippen derartig auslenkbar sind, während mindestens eine Anlagerippe fest und nicht auslenkbar an der Anlageseite der Auswerteeinheit vorgesehen ist. Eine mögliche Konstellation sieht beispielsweise drei Anlagerippen vor, von denen eine oder zwei an jeweils einem elastischen Ausleger angebracht sind, während die dritte Anlagerippe ortsfest an der Anlageseite vorgesehen ist. Auch eine Rippengestaltung mit zwei Anlagerippen, die beide auslenkbar sind, kann zweckmäßig sein.

Die Gestaltung mit Anlagerippen kann sich insbesondere auch als positiv im Kontext der magnetischen Anbringung der Auswerteeinheit am Gehäuse auswirken. Durch die Erstreckungsrichtung der Anlagerippen ist eine Verschieberichtung definiert, in Richtung derer die Auswerteeinheit gegenüber dem Gehäuse verschoben werden kann, um eine Trennung der Auswerteeinheit vom Gehäuse herbeizuführen.

Die Erfindung betrifft neben dem Spender mitsamt der Auswerteeinheit in seiner Gesamtheit auch die isolierte Auswerteeinheit, die zur lösbaren Anbringung an einem Spender in oben beschriebener Weise zur Erfassung der Nutzung des Spenders ausgebildet ist, insbesondere durch Verwendung eines Mikrofons und/oder eines Bewegungssensors.

Um in der oben beschriebenen Weise am Gehäuse eines Spenders angebracht zu werden, ist die Auswerteeinheit gemäß einer ersten Gestaltung im Bereich einer Anlageseite magnetisch oder magnetisierbar ausgebildet. Insbesondere kann mindestens ein Permanentmagnet an der Anlageseite vorgesehen sein, insbesondere ein Neodym-Magnet.

Gemäß einer zweiten Gestaltung weist die Auswerteeinheit mindestens zwei und vorzugsweise mindestens drei elastisch auslenkbare Haltearme auf, die das Gehäuse, insbesondere einen Speicherkörper des Gehäuses, bestimmungsgemäß umgreifen können und damit einen klemmenden Halt der Auswerteinheit am Gehäuse bewirken können.

Die Auswerteeinheit kann in oben bereits beschriebener Weise modular ausgestaltet sein und eine Befestigungseinheit sowie eine Elektronikeinheit aufweisen. Die Befestigungseinheit ist zur lösbaren Anbringung am Gehäuse ausgebildet und im Bereich der Anlageseite magnetisch oder magnetisierbar ausgebildet. Die Elektronikeinheit ist zur lösbaren Anbringung an der Befestigungseinheit vorgesehen und umfasst vorzugsweise die Gesamtheit der elektrischen/elektronischen Komponenten der Auswerteeinheit.

Vorzugsweise kann die Auswerteeinheit mehrere wechselbare Befestigungseinheiten umfassen, die an unterschiedliche Arten von Spendergehäusen angepasst sind und die wahlweise mit der Elektronikeinheit gekoppelt werden können.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 und 2 zeigen exemplarisch einen erfindungsgemäßen Flüssigkeitsspender mit einer erfindungsgemäßen Auswerteeinheit.
Fig. 3 bis 5 verdeutlichen eine erste Konfiguration der Ausgestaltung einer magnetischen Verbindung zwischen der Auswerteeinheit und einem Gehäuse des Flüssigkeitsspenders.
Fig. 6 bis 8 verdeutlichen eine zweite Konfiguration der Ausgestaltung einer magnetischen Verbindungzwischen der Auswerteeinheit und einem Gehäuse des Flüssigkeitsspenders.
Fig. 9 bis 11 verdeutlichen eine Ausgestaltung einer Verbindung zwischen der Auswerteeinheit und einem Gehäuse des Flüssigkeitsspenders durch an der Auswerteeinheit vorgesehene Haltearme.
Fig. 12 bis 15 zeigen eine mögliche modulare Ausgestaltung der Auswerteeinheit mit einer Befestigungseinheit und einer Elektronikeinheit sowie Kopplungsmittel zur Kopplung der Elektronikeinheit an die Befestigungseinheit.
Fig. 16 bis 18 verdeutlichen eine erste Konfiguration von Anlagerippen an einer Anlageseite der Auswerteeinheit.
Fig. 19 bis 21 verdeutlichen eine erste Konfiguration von Anlagerippen an einerAnlageseite der Auswerteeinheit.

### DETAILLIERTE BESCHREIBUNG DER AUSFÜHRUNGSBEISPIELE

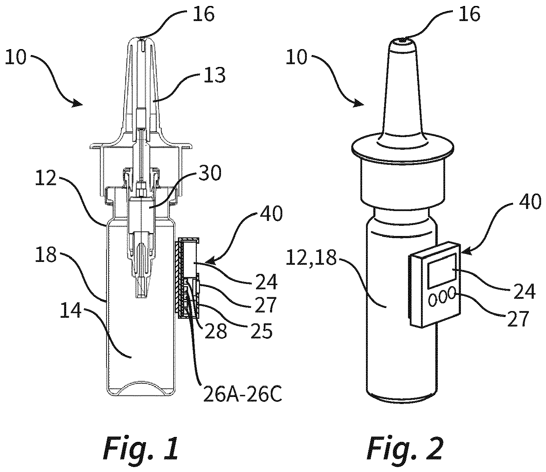

Fig. 1 und 2 zeigen einen erfindungsgemäßen Spender 10, der zum Austrag pharmazeutischer Flüssigkeiten ausgebildet ist. Der Flüssigkeitsspender 10 ist exemplarisch als Nasalspender ausgebildet und verfügt über ein Applikatorteil mit einem länglichen Nasalapplikator 13, an dessen distalem Ende eine Austragöffnung 16 vorgesehen ist. Der konkrete Spendertyp ist jedoch nur als Beispiel zu verstehen. Die Erfindung umfasst ebenso auch andere Spenderfür Medien und insbesondere Flüssigkeiten, insbesondere pharmazeutische Spender, die zur oralen oder topischen Abgabe von Medikamenten ausgebildet sind.

Durch Niederdrücken des Applikatorteils gegenüber einem Flüssigkeitsspeicher 14 bzw. dem den Flüssigkeitsspeicher bildenden Speicherkörper 18 kann eine Pumpeinrichtung 30 betätigt werden, die Flüssigkeit aus dem Flüssigkeitsspeicher 14 zur Austragöffnung 16 fördert.

Bei einem erfindungsgemäßen Spender 10 ist vorgesehen, dass die Nutzung des Spenders 10 durch den Anwender elektronisch erfasst wird. Hierfür ist eine elektronische Auswerteeinheit 40 vorgesehen. Diese umfasst im vorliegenden Beispiel eine Batterie 25, eine Anzeigeeinrichtung 24, eine Platine mit einem Prozessor 28, Bedienelemente 27 sowie verschiedene Sensoren 26A bis 26C.

Die Sensoren 26A-26C können insbesondere einen Bewegungssensor umfassen, der die Ausrichtung des Spenders und/oder Beschleunigungen erfasst. Weiterhin können die Sensoren 26A-26C ein Mikrofon umfassen, welches Geräusche erfasst, die bei der Handhabung des Spenders 10 auftreten. Die Auswertung der erfassten Daten erfolgt entweder direkt mittels des Prozessors 28 oder ganz oder teilweise auf einem externen System, welches über eine Funkschnittstelle mit der Auswerteeinheit 40 verbunden ist.

Exemplarisch kann der Prozessor 28 dafür ausgebildet sein, im Falle von mittels des Bewegungssensors erfassten Bewegungen des Spenders 10 das Mikrofon zu aktivieren. Mittels des Mikrofons können Geräusche erfasst werden, insbesondere durch das Spendergehäuse 12 übertragene Geräusche. Insbesondere gehen die verschiedenen Phasen eines Pumpvorgangs, also das Ausbringen von Flüssigkeit aus einer Pumpkammer und das erneute Ansaugen von Flüssigkeit aus dem Medienspeicher 14 in die Pumpkammer mit charakteristischen Geräuschen einher, die gut voneinander unterscheidbar sind.

Die Auswerteeinheit 40 ist am Speicherkörper 18 des Spendergehäuses 12 angebracht. Der Speicherkörper 18 weist eine zylindrische Grundform auf, vorliegend eine kreiszylindrische Grundform.

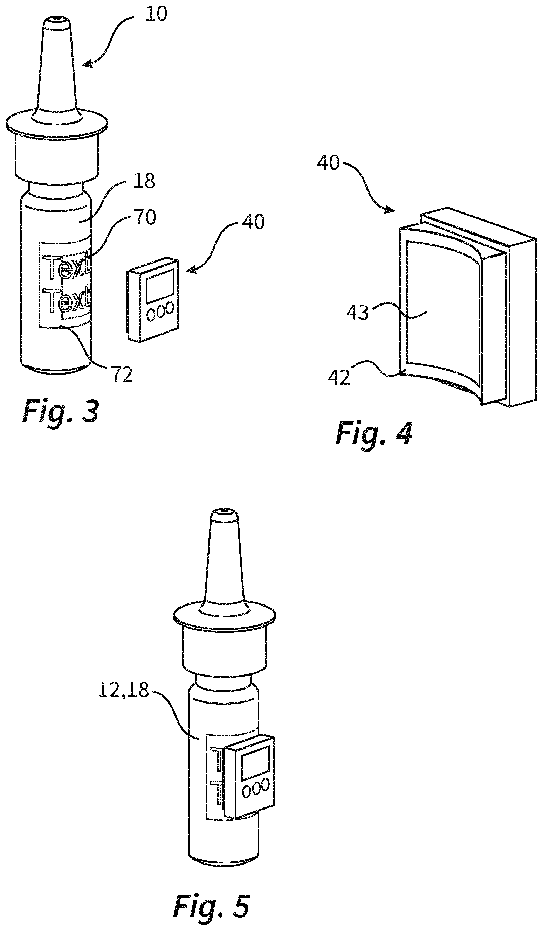

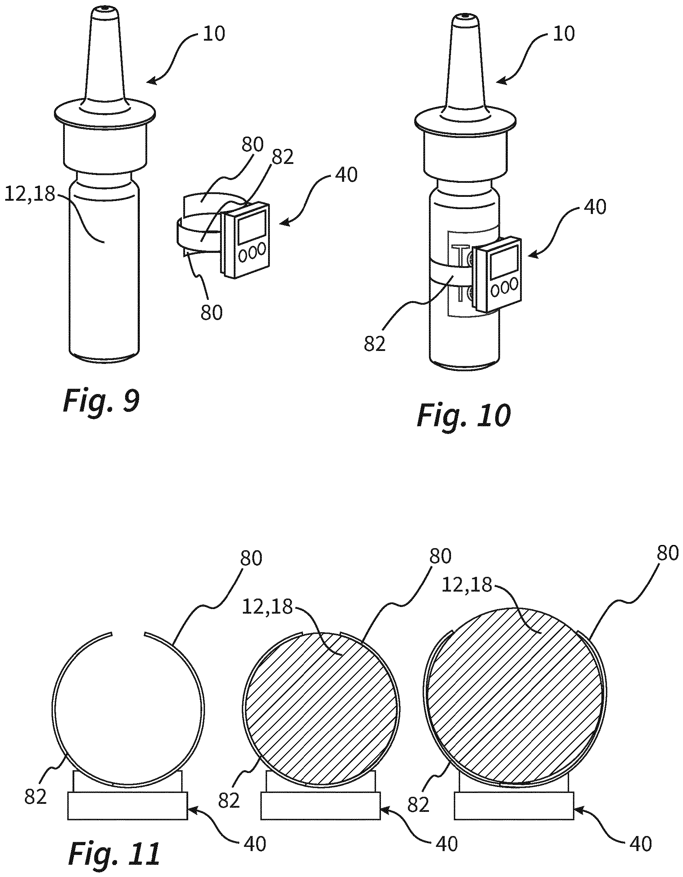

Zur Anbringung der Auswerteeinheit 40 am Speicherkörper 18 werden erfindungsgemäß zwei Befestigungsprinzipien vorgeschlagen, die anhand der Fig. 3 bis 8 bzw. anhand der Fig. 9 bis 11 vorgestellt werden.

Bei der Gestaltung der Fig. 3 bis 8 ist vorgesehen, dass die Auswerteinheit 40 magnetisch am Speicherkörper 18 befestigt ist.

Fig. 3 zeigt den Speicherkörper 18 des Spenders 10, der mit einem Etikett 72 versehen ist, welches beispielsweise die Produktbezeichnung oder Produktdetails enthalten kann oder aber auch Handhabungshinweise zur Anbringung der Auswerteeinheit 40. In das Etikett 72 integriert oder vom Etikett 72 überdeckt ist eine magnetische oder magnetisierbare Haltefläche 70 vorgesehen. Insbesondere kann es sich dabei um einen rechteckigen metallischen Blechabschnitt handeln.

Wie in Fig. 4 dargestellt ist, ist korrespondierend hierzu an einer Anlageseite 42 der Auswerteeinheit 40 ein Permanentmagnet 43 vorgesehen. Die Form und Größe der Haltefläche 70 und des Permanentmagneten 43 sind vorzugsweise derart aufeinander abgestimmt, dass die Auswerteinheit 40 in einer definierten Position am Speicherkörper 18 hält. Der so verbundene Zustand ist in Fig. 5 dargestellt.

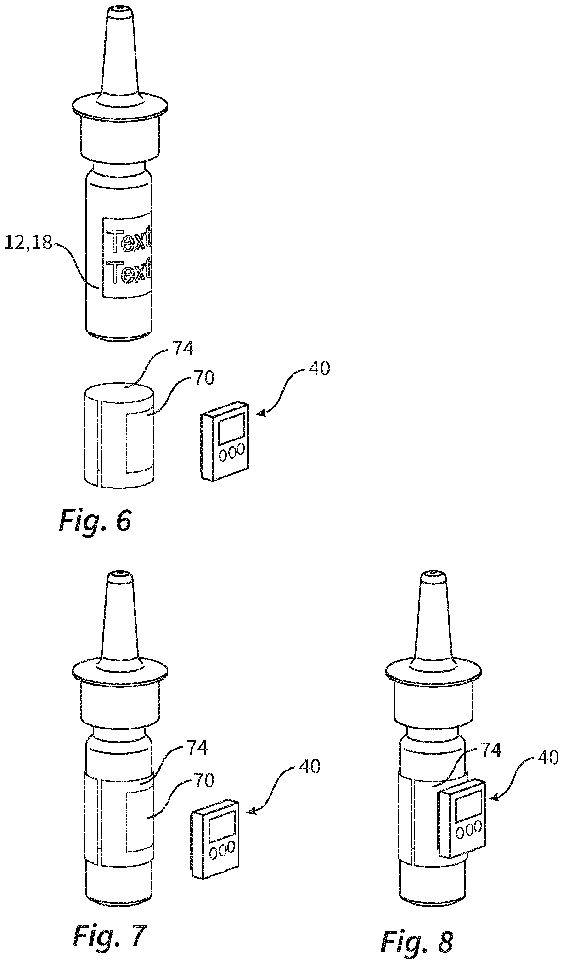

Fig. 6 zeigt eine Gestaltung, bei der eine Haltehülse 74 vorgesehen ist, die vollständig oder zumindest im Bereich einer Haltefläche 70 magnetisierbar ausgestaltet ist. Diese Haltehülse 74 ist als geschlitzte Hülse ausgebildet und kann in der in Fig. 7 verdeutlichten Weise auf den Speicherkörper 18 aufgeschoben werden, wobei der Innendurchmesser der Haltehülse 74 an den Außendurchmesser des Speicherkörpers 18 derart angepasst ist, dass die Haltehülse 74 nach dem Aufschieben auf den Speicherkörper 18 elastisch aufgeweitet ist und hierdurch klemmend am Speicherkörper 18 hält.

Die Haltefläche 70 als Teil der Haltehülse gestattet in der in Fig. 8 verdeutlichten Weise die magnetische Anbringung der Auswerteeinheit 40.

Die Fig. 9 bis 11 zeigen eine andere Form der Befestigung einer Auswerteeinheit 40 am Speicherkörper 18. Wie anhand der Fig. 9 ersichtlich ist, ist bei dieser Gestaltung vorgesehen, dass am Gehäuse der Auswerteeinheit insgesamt drei Haltearme 80, 82 vorgesehen sind, die jeweils eine gekrümmte Form aufweisen und gemeinsam einen Bereich zur Aufnahme des Speicherkörpers 18 umgeben. Der obere und der untere Haltearm 80 erstrecken sich vom Gehäuse der Auswerteeinheit 40 gegen den Uhrzeigersinn. Der dazwischenliegende Haltearm 82 erstreckt sich um das Gehäuse der Auswerteeinheit 40 aus im Uhrzeigersinn.

Im nicht angebrachten Zustand der Fig. 9 sind die Haltearme 80, 82 nicht elastisch ausgelenkt. Wird jedoch der Speicherkörper 18 in den von den Haltearmen umgebenen Bereich einschoben, so werden die Haltearme hierdurch leicht auseinander gedrückt, so dass sie klemmend an der Außenseite des Speicherkörpers anliegen und hierdurch eine sichere Kopplung schaffen. Der montierte Zustand ist in Fig. 10 dargestellt.

Fig. 11 verdeutlicht, wie die Auswerteeinheit an Gehäusen 12 unterschiedlichen Durchmessers angebracht werden kann. Die linke Darstellung der Fig. 11 zeigt die Auswerteeinheit 40 im nicht genutzten Zustand. Die mittlere Darstellung der Fig. 11 zeigt die Auswerteeinheit 40 nach Ankopplung an ein Gehäuse 12, dessen Außendurchmesser nur geringfügig größer als der Ruhezustand der Haltearme 80, 82 ist. Die Haltearme 80, 82 sind hierdurch elastisch nach außen ausgelenkt und klemmen den Speicherkörper 18 daher ein. Die Darstellung rechts in Fig. 11 zeigt die Verwendung der Auswerteeinheit40 mit einem bedeutend größeren Gehäuse 12. Die Haltearme 80, 82 sind hier deutlich weiter aufgeweitet. Hierdurch bedingt liegen sie nicht über ihre gesamte Länge an der Außenseite des Gehäuses 12 an. Da jedoch die Auslenkung und hiermit einhergehend die Rückstellkraft erheblich größer ist, wird dennoch ein sicherer Halt auch an diesem Gehäuse 12 erzielt.

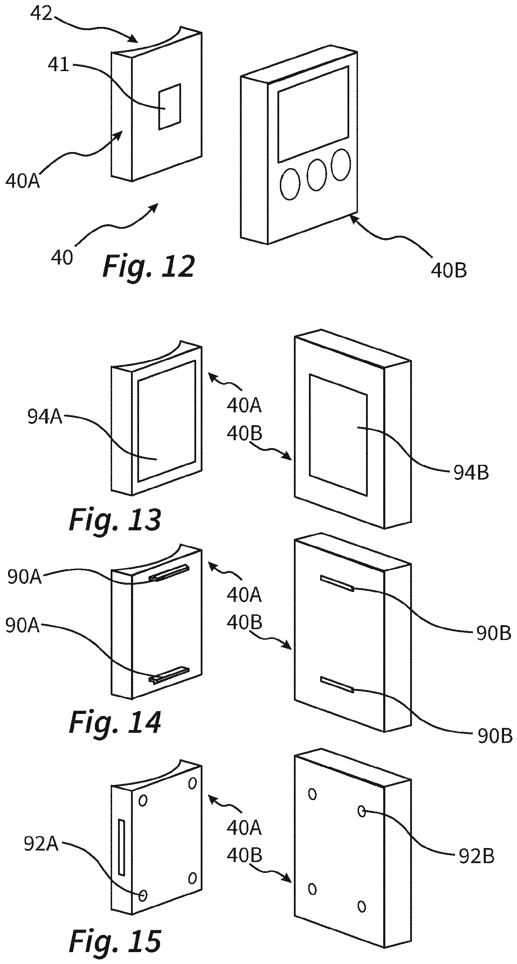

Die Fig. 12 bis 15 verdeutlichen die Möglichkeit, dieAuswerteeinheit40 modularzu gestalten. In nicht näher dargestellter Weise kann die Anlageseite 12 der Auswerteeinheit 40 entsprechend den Fig. 3 bis 8 magnetisch oder magnetisierbar ausgestaltet sein oder mit Haltearmen entsprechend der Fig. 9 bis 11 versehen sein.

Wie anhand der Fig. 12 verdeutlicht, untergliedert sich die Auswerteeinheit 40 in solchen Fällen in eine Befestigungseinheit 40A, an der die Anlageseite 42 vorgesehen ist, sowie in eine Elektronikeinheit 40B, die die überwiegende Zahl an elektrischen/elektronischen Komponenten aufweist, vorzugsweise die Gesamtheit der elektronischen Komponenten. Es kann allerdings vorgesehen sein, dass die Befestigungseinheit 40A über ein elektronisches Identifikationsmittel wie einen RFID-Chip 41 verfügt, damit die Elektronikeinheit 40B erkennen kann, ob sie mit einer Befestigungseinheit 40A verbunden ist bzw. mit welcher Befestigungseinheit 40A sie verbunden ist.

Die Teileinheiten 40A, 40B sind vorzugsweise werkzeuglos miteinander koppelbar. Bei der Gestaltung der Fig. 13 sind die beiden Teileinheiten mit magnetischen oder magnetisierbaren Kopplungsflächen 94A, 94B verbunden. Bei einersolchen Gestaltung ist es möglich, einen gemeinsamen Magneten an der Befestigungseinheit 40A vorzusehen, der der Kopplung an das Gehäuse 12 sowie der Kopplung mit der Elektronikeinheit 40B dient.

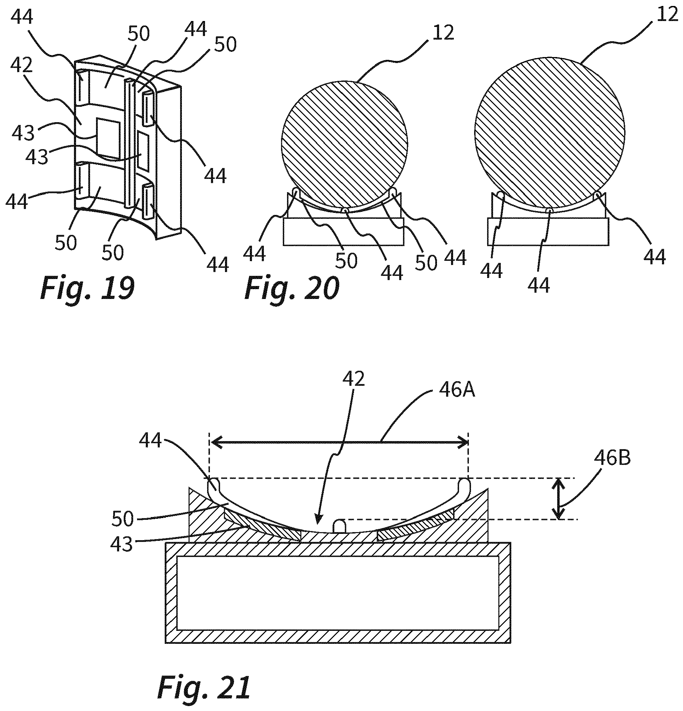

Bei der Gestaltung der Fig. 13 sind Rastlaschen 90A an der Befestigungseinheit 40Avorgesehen, die in Rastöffnungen 90B an der Etektronikeinheit 40B verrasten.Vorzugsweise sind die Rastlaschen 90A abweichend von der Gestaltung der Fig. 20 manuell auslenkbar, um die Verbindung wieder trennen zu können. Es kann jedoch auch vorgesehen sein, dass die Verbindung der Elektronikeinheit 40B mit der Befestigungseinheit 40A bestimmungsgemäß unlösbar ist, so dass eine entsprechende Kopplung nur einmalig möglich ist, insbesondere bei einer bereits im Zuge der Herstellung stattfindenden Festlegung auf eine bestimmte Befestigungseinheit 40A.

Bei der Gestaltung der Fig. 14 sind Schraublöcher 92A, 92B an der Elektronikeinheit 40B und der Befestigungseinheit 40A vorgesehen, durch die hindurch die beiden Teileinheiten miteinander verschraubt werden können. Diese Art der Verbindung ist üblicherweise nicht dafür vorgesehen, vom Endanwender gelöst zu werden, sondern dient dem Zweck, im Zuge der Herstellung eine einheitliche Elektronikeinheit 40B dauerhaft mit einer Befestigungseinheit 40A verbinden zu können.

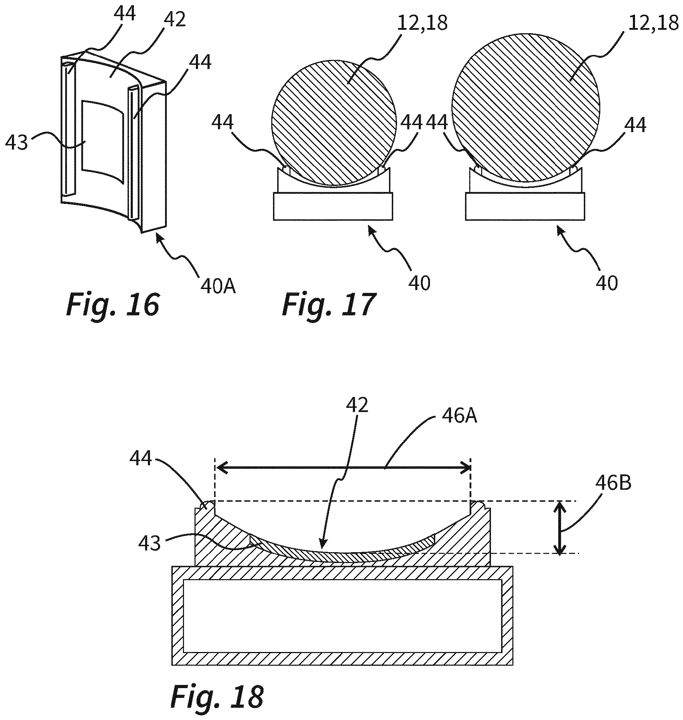

Die Fig. 16 bis 21 verdeutlichen, dass es von Vorteil sein kann, wenn die Auswertungseinheit 40 bzw. ihre Befestigungseinheit 40A mit Anlagerippen 44 versehen ist. Die zur Anlage am Gehäuse 12 bzw. der Haltehülse 74 vorgesehen sind.

Die Fig. 16 bis 21 zeigen eine erste Konfiguration von Anlagerippen 44. In Fig. 16 ist die Rückseite der Auswerteeinheit 40 dargestellt. Diese Rückseite bildet eine Anlageseite 42, wobei zwei parallele Anlagerippen 44 zueinander ortsfest an derAnlageseite 42 vorgesehen sind. Zwischen den beiden Anlagerippen 44 ist ein zurückgesetzter Zwischenbereich vorgesehen, wobei hier ein Permanentmagnet 43 befestigt ist. Wie sich anhand der Fig. 17 ersehen lässt, ermöglichen es die Anlagerippen 44, an Spendergehäusen 12 unterschiedlicher Krümmungsradien anzuliegen. Die gleiche Auswerteeinheit 40 bzw. die gleiche Befestigungseinheit 40A der Auswerteeinheit 40 kann also für unterschiedliche Spender 10 gleichermaßen gut verwendet werden.

Fig. 18 verdeutlicht nochmals die Geometrie der Anlageseite 42. Es ist zu ersehen, dass diese eine grundsätzlich gewölbte konkave Form aufweist und dass die beiden Anlagerippen 44 sich über diese gewölbte konkave Form erheben. Der Abstand 46A entspricht der Breite des Zwischenbereichs. Der Abstand 46B zeigt an, wieweit der Zwischenbereich maximal gegenüber den Anlagerippen 44 zurückgesetzt ist. Da im Zwischenbereich auch der Permanentmagnet 43 angeordnet ist, ist diese aufgrund der Anlagerippen 44 etwas von der Außenseite des Spendergehäuses 12 beabstandet. Bei Wahl von ausreichend starken Magneten, insbesondere Neodym-Magneten, steht dieser Abstand einer ausreichenden Haltekraft jedoch nicht entgegen.

Die Fig. 19 bis 21 zeigen eine zweite Konfiguration von Anlagerippen 44. Wie anhand der Fig. 19 zu ersehen ist, ist die Anlageseite 42 hier mit insgesamt drei Anlagerippen 44 versehen. Eine zentrische Anlagerippe 44 ist fest mit dem Gehäuse der Auswerteeinheit 40 verbunden und gegenüber diesem nicht in relevantem Maße auslenkbar. Zwei seitliche Anlagerippen 44 sind dagegen aus Auslegern 50 vorgesehen, wobei beide seitliche Anlagerippen 44 jeweils aus zwei miteinander fluchtenden Rippensegmenten bestehen, die ihrerseits jeweils an einem Ausleger 50 vorgesehen sind. Die beiden äußeren Anlagerippen können daher federnd ausgelenkt werden.

Die Ausleger 50 gestatten es, die außenliegenden Anlagerippen 44 gegen die Federkraft der Ausleger 50 relativ zueinander zu verlagern. Hierdurch kann trotz insgesamt dreier Anlagerippen erreicht werden, dass alle Anlagerippen bei VerwendungderAuswerteeinheit40 mit unterschiedlichen Spendergehäusen 12 bzw. Speicherkörpern 18 jeweils am Spendergehäuse 12 anliegen. Bei Spendergehäusen 12/Speicherkörpern 18 mit größeren Durchmessern werden die Ausleger50 elastisch ausgelenkt.

## Patentansprüche

1. Spender (10) zum Austrag pharmazeutischer oder kosmetischer Medien mit den folgenden Merkmalen:
a. der Spender (10) weist ein Gehäuse (12) auf, und
b. der Spender (10) weist einen Medienspeicher (14) auf, und
c. der Spender (10) weist eine Austragöffnung (16) auf, durch die Medium aus dem Medienspeicher (14) in eine umgebende Atmosphäre abgegeben werden kann, und
d. der Spender (10) weist eine elektronische Auswerteeinheit (40) zur Erfassung der Nutzung des Spenders auf, und
e. die elektronische Auswerteeinheit (40) ist zur lösbaren Anbringung am Gehäuse (12) des Spenders (10) ausgebildet,
**gekennzeichnet durch** die folgenden weiteren Merkmale:
f. an der Außenseite des Gehäuses (12), insbesondere an einem Speicherkörper (18) des Gehäuses (12), ist eine magnetische oder magnetisierbare Haltefläche (70) vorgesehen, und
g. eine Anlageseite (42) der Auswerteeinheit (40) ist hierzu korrespondierend magnetisch oder magnetisierbar ausgebildet, so dass die Auswerteeinheit (40) magnetisch am Gehäuse (12) befestigt werden kann.

2. Spender (10) nach Anspruch 1 mit dem folgenden weiteren Merkmal:
a. die magnetische oder magnetisierbare Haltefläche (70) ist durch ein beschriftetes Etikett (72) überdeckt oder in ein beschriftetes Etikett integriert,
insbesondere mit mindestens einem der folgenden zusätzlichen Merkmale:
b. die Beschriftung umfasst eine Produktbezeichnung und/oder eine Produktbeschreibung, und/oder
c. die Beschriftung umfasst eine Handhabungsanleitung betreffend die Anbringung der Auswerteeinheit.

3. Spender (10) nach Anspruch 1 oder 2 mit dem folgenden weiteren Merkmal:
a. die magnetische oder magnetisierbare Haltefläche (70) wird durch eine am Gehäuse klemmend befestigte Haltehülse (74) oder Halteklemme gebildet.

4. Spender (10) nach einem der vorstehenden Ansprüche mit dem folgenden weiteren Merkmal:
a. die magnetische oder magnetisierbare Haltefläche (70) ist klebend am Gehäuse befestigt.

5. Spender (10) zum Austrag pharmazeutischer oder kosmetischer Medien mit den folgenden Merkmalen:
a. der Spender (10) weist ein Gehäuse (12) auf, und
b. der Spender (10) weist einen Medienspeicher (14) auf, und
c. der Spender (10) weist eine Austragöffnung (16) auf, durch die Medium aus dem Medienspeicher (14) in eine umgebende Atmosphäre abgegeben werden kann, und
d. der Spender (10) weist eine elektronische Auswerteeinheit (40) zur Erfassung der Nutzung des Spenders auf, und
e. die elektronische Auswerteeinheit (40) ist zur lösbaren Anbringung am Gehäuse (12) des Spenders (10) ausgebildet,
**gekennzeichnet durch** das folgende weitere Merkmal:
f. die Auswerteeinheit (40) weist mindestens zwei und vorzugsweise mindestens drei elastisch auslenkbare Haltearme (80, 82) auf, die das Gehäuse (12), insbesondere einen Speicherkörper (18) des Gehäuses, umgreifen und damit einen klemmenden Halt der Auswerteinheit (40) am Gehäuse bewirken.

6. Spender (10) nach Anspruch 5 mit dem folgenden weiteren Merkmal:
a. es sind mindestens drei Haltearme (80, 82) vorgesehen, von denen zwei Haltearme (80) sich in einer ersten Erstreckungsrichtung erstrecken und von denen der dritte Haltearm (82) zwischen diesen beiden Haltearmen (80) angeordnet ist und sich in entgegengesetzter Erstreckungsrichtung erstreckt.

7. Spender (10) nach Anspruch 5 oder 6 mit dem folgenden weiteren Merkmal:
a. die Haltearme erstrecken sich kreisabschnittsförmig,
vorzugsweise mit mindestens einem der folgenden zusätzlichen Merkmale:
b. mindestens ein Haltearm überspannt einen Winkelbereich von mindestens 120°, und/oder
c. ein Durchmesser des Grundkreises der Kreisabschnittsform beträgt zwischen 10 mm und 25 mm.

8. Spender (10) nach einem der vorstehenden Ansprüchen mit den folgenden weiteren Merkmalen:
a. die Auswerteeinheit (40) umfassteine Befestigungseinheit (40A) und eine Elektronikeinheit (40B), und
b. die Befestigungseinheit (40A) ist zur lösbaren Anbringung am Gehäuse (12) ausgebildet, und
c. die Elektronikeinheit (40B) ist zur lösbaren Anbringung an der Befestigungseinheit (40A) vorgesehen.

9. Spender (10) nach Anspruch 8 mit mindestens einem der folgenden zusätzlichen Merkmale:
a. die Befestigungseinheit (40A) ist zur werkzeuglos lösbaren Anbringung am Gehäuse (12) ausgebildet, und/oder
b. die Elektronikeinheit (40B) ist magnetisch an der Befestigungseinheit (40A) befestigt, und/oder
c. die Elektronikeinheit (40B) ist durch einen Rastmechanismus mit einer elastisch auslenkbaren Rastlasche (90) an der Befestigungseinheit (40A) befestigt, und/oder
d. die Befestigungseinheit (40A) weist ein Identifikationsmerkmal auf, insbesondere abgelegt in einem RFID-Chip der Befestigungseinheit, welcherdurch einen Sensor (26B) der Elektronikeinheit (40B) auslesbar ist.

10. Spender (10) zum Austrag pharmazeutischer oder kosmetischer Medien mit den folgenden Merkmalen:
a. die elektronische Auswerteeinheit (40) weist eine Anlageseite (42) auf, die im angebrachten Zustand der Auswerteeinheit (40) in Richtung des Gehäuses (12) weist, und
b. die elektronische Auswerteeinheit (40) weist an der Anlageseite (42) zwei zueinander parallele Anlagerippen (44) zur Anlage am Gehäuse (12) auf, insbesondere zur Anlage an einem Speicherkörper (18) des Medienspeichers (14).

11. Spender (10) nach Anspruch 10 mit mindestens einem der folgenden weiteren Merkmale:
a. die Rippen (44) weisen einen Abstand (46A) voneinander von mindestens 8 mm auf, und/oder
b. die Rippen (44) erheben sich gegenüber einem zwischen ihnen liegenden Zwischenbereich (48) um einen Abstand (46B) von mindestens 1,5 mm und/oder
c. mindestens eine Rippe (44) ist an einem elastischen Ausleger (50) angebracht, und/oder
d. mindestens eine Rippe (44) wird durch mehrere zueinander fluchtende Rippensegmente gebildet, die vorzugsweise jeweils an einem elastischen Ausleger (50) vorgesehen sind.

12. Spender (10) nach einem der vorstehenden Ansprüche mit mindestens einem der folgenden weiteren Merkmale:
a. die Auswerteeinheit (40) umfasst mindestens einen Sensor (26A, 26B, 26C), insbesondere einen Bewegungssensor (26A) und/oder ein Mikrofon (26C), und/oder
b. die Auswerteeinheit (40) umfasst eine Ausgabeeinrichtung (24), insbesondere in Form mindestens einer LED, eines Displays (24), eines Vibrators und/oder eines Lautsprechers,
c. die Auswerteeinheit umfasst ein Funkmodul (22), insbesondere ein Bluetooth- und/oder WIFI-Funkmodul (22), und/oder
d. die Auswerteeinheit umfasst eine Energiequelle (25), insbesondere in Form einer Batterie.

13. Spender (10) nach einem der vorstehenden Ansprüche mit dem folgenden weiteren Merkmal:
a. der Spender (10) ist als Flüssigkeitsspender (10) ausgebildet, wobei der Medienspeicher (14) als Flüssigkeitsspeicher (14) ausgebildet ist,
insbesondere mit mindestens einem der folgenden zusätzlichen Merkmale:
b. der Spender ist als Quetschflaschenspender ausgebildet und weist einen elastisch verformbaren Speicherkörper des Flüssigkeitsspeichers auf, durch dessen manuelle Komprimierung Flüssigkeit ausgetragen werden kann, oder
c. der Spender (10) istals Pumpspenderausgebildet, der über eine manuell betätigbare Pumpeinrichtung (30) verfügt, die Flüssigkeit aus dem Flüssigkeitsspeicher (14) zur Austragöffnung (16) fördert, und/oder
d. der Spender (10) ist als Sprühspender ausgebildet, und/oder
e. der Spender ist als Inhalator ausgebildet, insbesondere als Spender mit einem zur Abgabe einer Dosis der Flüssigkeit niederdrückbarem Container,
f. der Spender ist als Tropfenspender ausgebildet, insbesondere mit einer die Austragöffnung umgebenden Tropfenbildungsfläche, und/oder
g. der Spender ist als Nasalspender ausgebildet, insbesondere mit einem langgestreckten Nasalapplikator, an dessen distalem Ende die Austragöffnung vorgesehen ist.

14. Auswerteeinheit füreinen Spender nach einem dervorstehenden Ansprüche mit den folgenden Merkmalen:
a. die Auswerteeinheit (40) ist zur lösbaren Anbringung an einem Spender (10) ausgebildet, und
b. die elektronische Auswerteeinheit (40) ist zur Erfassung der Nutzung des Spenders ausgebildet und weist hierfür mindestens einen Sensor (26A, 26C) auf,
**gekennzeichnet durch** mindestens eines der folgenden weiteren Merkmale:
c. eine Anlageseite (42) der Auswerteeinheit (40) ist magnetisch oder magnetisierbar ausgebildet, so dass die Auswerteeinheit (40) magnetisch am Gehäuse (12) befestigt werden kann, und/oder
d. die Auswerteeinheit (40) weist mindestens zwei und vorzugsweise mindestens drei elastisch auslenkbare Haltearme (80, 82) auf, die das Gehäuse (12), insbesondere einen Speicherkörper (18) des Gehäuses, umgreifen und damit einen klemmenden Halt der Auswerteinheit (40) am Gehäuse bewirken.

15. Auswerteeinheit gemäß Anspruch 14 mit dem folgenden zusätzlichen Merkmal:
a. dieAuswerteeinheit (40) umfassteine Befestigungseinheit (40A) und eine Elektronikeinheit (40B), wobei die Befestigungseinheit (40A) zur lösbaren Anbringung am Gehäuse (12) ausgebildet ist und wobei die Elektronikeinheit (40B) zur lösbaren Anbringung an der Befestigungseinheit (40A) vorgesehen ist, vorzugsweise mit dem folgenden zusätzlichen Merkmal:
b. die Auswerteeinheit (40) umfasst eine Mehrzahl von Befestigungseinheiten (40A), die wahlfrei an der Elektronikeinheit (40B) angebracht werden können.
